Europäisches Patentamt

European Patent Office

Office européen des brevets

(11) Veröffentlichungsnummer: **0 456 015 A1**

## (12) EUROPÄISCHE PATENTANMELDUNG

(21) Anmeldenummer: 91106193.5

(22) Anmeldetag: 18.04.91

(51) Int. Cl.⁵: **A61B 17/22**

(30) Priorität: 10.05.90 DE 4014956

(43) Veröffentlichungstag der Anmeldung:
**13.11.91 Patentblatt 91/46**

(84) Benannte Vertragsstaaten:
**DE FR GB IT**

(71) Anmelder: **Schott Glaswerke**
**Hattenbergstrasse 10**
**W-6500 Mainz(DE)**
(84) **DE FR IT**

Anmelder: **Carl-Zeiss-Stiftung trading as**
**SCHOTT GLASWERKE**
**Hattenbergstrasse 10**
**W-6500 Mainz 1(DE)**
(84) **GB**

(72) Erfinder: **Schönborn, Karl-Heinz, Dr.**
**Konrad-Adenauer-Strasse 27**
**W-6500 Mainz 41(DE)** ·

(54) Distale Schutzkappe für einen Laser-Faser-Katheter.

(57) Es wird eine distale Schutzkappe (1) für einen Laser-Faser-Katheter (7) zur Entfernung von biologischem Material beschrieben, die aus einem im wesentlichen zylindrischen Körper (2) aus einem für die Laserstrahlung transparenten Material besteht. Der zylindrische Körper, der am Katheterende derart befestigt ist, daß er mit seiner proximalen Stirnfläche (5) die distalen Faserendflächen abdeckt und seine distale Stirnfläche (4) eine Austrittsoberfläche für die Strahlung festlegt, besteht nach der Erfindung aus Glas oder Glaskeramik und ist an seiner Außenfläche mit einer Reflexionsschicht (3) versehen, welche die in die proximale Stirnfläche der Kappe eintretende Strahlung am seitlichen Austritt aus der Schutzkappe hindert.

Figur 1

EP 0 456 015 A1

Die Erfindung betrifft eine distale Schutzkappe für einen Laser-Faser-Katheter nach dem Oberbegriff des Patentanspruchs 1.

Es ist bekannt, daß sich bestimmte Ablagerungen in Gefäßsystemen, beispielsweise des menschlichen Körpers, insbesondere in Adern, durch Einwirkung von Laserstrahlung bestimmter Energiedichte, wie vermutet wird, infolge des photohydraulischen Effektes ablösen und sich die behandelten Gefäße somit rekanalisieren lassen. Für die Übertragung der Laserstrahlung in die zu behandelnden Gefäßbereiche sind Katheter mit integrierten optischen Fasern vorgesehen, die in die Gefäße eingeführt und, da die Energiedichte unmittelbar am Austrittsende der Faser am größten ist, so weit vorgeschoben werden, bis das freie Austrittsende der Faser beziehungsweise des Faserbündels unmittelbar an dem Gefäßverschluß zur Anlage kommt. Da in dem Bereich zwischen dem Austrittspunkt des Laserstrahls am Ende der Faser bzw. des Faserbündels und dem zu beseitigenden Verschluß immer organisches Material, beispielsweise Blut, vorhanden ist, kann bei der Beseitigung der Ablagerungen das Problem auftreten, daß dieses Material an dem Faserende verklebt und verkohlt, wodurch es zu einer Überhitzung und Zerstörung der optischen Faser kommen kann.

Eine weitere Schwierigkeit besteht darin, daß die freien Faserenden auch durch den photohydraulischen Effekt, der auf der Stoßwellenerzeugung durch den Ablationsprozeß beruht, beschädigt werden können.

Um die distalen Endflächen der Fasern gegen die oben beschriebenen mechanischen und thermischen Belastungen zu schützen, wird in der Patentliteratur (z.B. EP-OS 0 195 375, DE-OS 37 39 965 A1) vorgeschlagen, das distale Ende eines Katheters mit einer Schutzkappe zu versehen, welche die Faserendflächen abdeckt und den Katheter distal abschließt. Die Schutzkappe besteht aus einem für die Laserstrahlung hochtransparenten Material, so daß die Strahlung die Abdeckung ungehindert durchdringen und aus dem distalen Ende des Katheters austreten kann.

Im Allgemeinen besitzen die Katheter einen Katheterkörper mit einem annähernd kreisförmigen Querschnitt. Die Schutzkappe ist an die Gestalt des Katheterkörpers angepaßt und besitzt in der Regel die Form eines Zylinders.

Die optischen Fasern in dem Katheter sind an ihrem distalen Ende entweder mit einem geeigneten Bindemittel miteinander verklebt, oder aber mittels eines Faserhalters, z.B. eines Metallzylinders mit Bohrungen zur Aufnahme der Fasern, fixiert. Die i.a. flach polierte distale Stirnfläche der Fasern bzw. der Kombination Fasern/Faserhalter kann an der proximalen Stirnfläche der Kappe direkt anliegen bzw. mittels eines geeigneten optisch transparenten Bindemittels mit dieser verklebt sein, oder aber, wie in der EP-OS 0 195 375 beschrieben, zu dieser beabstandet angeordnet sein, wobei der Zwischenraum zwischen den Faserendflächen und der proximalen Stirnfläche der Kappe mit einem geeigneten Medium, z.B. einem inerten Gas oder einem festen oder flüssigen Material mit z.B. angepaßter Brechzahl, ausgefüllt sein kann. Der Abstand zwischen der distalen Endfläche der Fasern und der distalen Austrittsoberfläche der Schutzkappe ist i.a. so gewählt, daß die von den Faserendflächen ausgehenden Strahlungskegel an der Austrittsoberfläche einander gerade überlappen. Eine weitere Vergrößerung des Abstandes ist nicht sinnvoll, da dies zu einer unnötigen Verlängerung des starren Katheterendes führt.

Seit neuerer Zeit sind Katheter bekannt, die aus den verschiedensten Gründen ein zentrales Lumen aufweisen, z.B. zur Aufnahme eines Führungsdrahtes oder zum Zu- und Abführen einer Spühlflüssigkeit, usw. Bei diesen Kathetern sind die Fasern z.B. auf einem Kreisring um das zentrale Lumen angeordnet. Die distalen Schutzkappen für diese Katheter entsprechen im wesentlichen denen für Katheter ohne Lumen, sie weisen im Gegensatz zu diesen aber eine zentrale Bohrung auf, in die sich das Lumen fortsetzt.

Das Lumen kann sowohl einen annähernd kreisförmigen als auch einen von der Kreisform abweichenden Querschnitt, beispielsweise, wie aus der DE-OS 36 10 270 A1 bekannt, einen lappenförmigen Querschnitt besitzen. Die Gestalt der Bohrung kann an die des Lumens angepaßt sein.

Die in der EP-OS 195 375 beschriebene Schutzkappe besteht im wesentlichen aus einem Schutzfenster aus Glas, Quarzglas, Saphir oder einem anderen, für die verwendete Laserstrahlung transparenten Material. Das Fenster, das sich auf der dem Katheter zugewandten Seite in einem ringförmigen Abschnitt fortsetzt, kann als flache Scheibe geformt, aber auch sphärisch gekrümmt, beispielsweise als Linse ausgebildet sein.

Ein Problem stellt die Befestigung des Fensters am distalen Katheterende dar. Nach der Durckschrift ist vorgesehen, das Fenster lediglich am Rand des ringförmigen Abschnitts mit dem Katheterkörper zu verbinden. Dabei können Bindemittel, z.B. ein Epoxydkleber, eingesetzt werden. Ebenso ist es möglich, das Fenster durch Aufschrumpfen des Katheterschlauches zu befestigen.

Es ist zu erwarten, daß ein wie oben beschrieben befestigtes Fenster größeren mechanischen Belastungen, beispielsweise den bei dem Ablationsprozeß auftretenden Stoßwellen, nicht standhält und sich vom Katheterkörper löst.

Ein weiterer Nachteil besteht darin, daß die Strahlung, die sich nach Austritt aus den Faserendflächen noch innerhalb des Katheterendes auf einem Strahlungskegel ausbreitet, seitlich aus der Kappe austritt, wodurch ein nicht unerheblicher Anteil an Strahlungsleistung verlorengeht.

Aus der US-PS 3 858 577 ist bekannt, ein Schutzfenster aus Glas, Quarzglas oder Saphir mittels einer Außenhülse aus Edelstahl, welche sich über eine bestimmte Länge über die die Laserstrahlung übertragende Faser erstreckt, am distalen Faserende zu befestigen, wobei es in der Glasfasertechnik üblich ist, die Verbindung zwischen derartigen Hülsen und Glaskörpern durch mechanisches Verpressen oder Verkleben herzustellen.

Nachteilig an diesem Katheterende ist, daß auch hier im Bereich des Fensters infolge der Strahlaufweitung Strahlung seitlich aus der Kappe austritt und in der Hülse bzw. in der Verklebung teilweise absorbiert wird, was zur Beschädigung des distalen Katheterendes führen kann.

Aus der DE-OS 37 39 965 A 1 ist bekannt, sowohl die die distalen Faserenden abdeckende Schutzkappe als auch die diese umgebende Befestigungshülse aus einem temperaturbeständigen Glas zu fertigen. Beide sind fest miteinander verbunden und bilden einen im wesentlichen zylindrischen Körper, der zwei miteinander im Eingriff stehende axiale Bohrungen mit unterschiedlichen Durchmessern aufweist, in die sich u.a. das Katheterlumen fortsetzt. Die Bohrung mit dem größeren Durchmesser dient darüber hinaus zur Aufnahme des Katheterendes mit den z.B. mittels eines Faserhalters fixierten distalen Faserenden, der relativ dickwandige Bereich mit der kleineren Bohrung stellt das die Fasern abdeckende Schutzfenster dar. Die Schutzkappe kann nach der Druckschrift z.B. aus zwei Hülsen hergestellt werden, wobei eine kurze Hülse mit kleinerem Durchmesser in den Endbereich eines länglichen Glaszylinders mit größerem Durchmesser eingebracht und dort eingeschmolzen wird. Wahlweise kann die kurze Hülse, die das eigentliche Schutzfenster darstellt auch aus Saphir gefertigt sein. Die Verwendung von Schutzfenstern aus Saphir ist an sich bekannt. Dieses Material zeichnet sich nicht nur durch eine hohe Transparenz im interessierenden Wellenlängenbereich, sondern auch durch eine hohe Temperaturfestigkeit und eine große Härte aus. Darüber hinaus ist Saphir polierfähig, so daß die distale Austrittsfläche zur Vermeidung von Rückreflexionen in das distale Katheterende als stark reflektierende Fläche ausgebildet werden kann.

Nachteilig an der oben beschriebenen Schutzkappe ist, daß bei Verwendung von Hülse und Fenster aus Glas, wie bereits oben beschrieben, die Strahlung seitlich aus der Kappe in die Umgebung austreten kann. Bei Verwendung eines Saphirfensters in einer Glashülse wird zwar die Strahlung in der Regel sicher zur Austrittsoberfläche des Fensters geführt; diese Ausführungsform hat jedoch den Nachteil, daß zum einen Saphir ein sehr kostspieliges und aufgrund seiner Härte nur aufwendig bearbeitbares Material ist, und daß zum anderen eine stabile Verbindung zwischen dem Fenster und der Hülse nur sehr schwierig herzustellen ist. Ein weiterer Nachteil besteht noch darin, daß durch das Einschmelzen der Saphirhülse in die Glashülse mechanische Spannungen auftreten können, die zum Bruch der Glashülse führen können. Bei einer Verklebung treten die zuvor beschriebenen Schwierigkeiten durch Absorption der Strahlung in der Verklebung auf, wird dagegen die Glashülse auf das Saphirfenster aufgeschrumpft, kann es aufgrund des unterschiedlichen Wärmeausdehnungsverhaltens der beiden Materialien bei thermischer Belastung zur Ausbildung von Rissen im Glaskörper kommen.

Die Aufgabe der Erfindung besteht darin, eine distale Schutzkappe für einen Laser-Faser-Katheter bereitzustellen, welche die aus dem Stand der Technik bekannten Nachteile nicht aufweist.

Diese Aufgabe wird mit einer distalen Schutzkappe mit den im Patentanspruch 1 beschriebenen Merkmalen gelöst.

Die erfindungsgemäße Schutzkappe besteht aus einem im wesentlichen zylindrischen Körper aus Glas, bevorzugt Kieselglas, oder falls eine noch geringere thermische Ausdehnung der Schutzkappe gewünscht ist, aus Glaskeramik. An der Zylinderaußenfläche ist der Körper mit einer dünnen Reflexionsschicht überzogen, um den seitlichen Austritt von Strahlung aus dem Zylinder zu verhindern.

Für einen Katheter mit einem zentralen Lumen weist der zylindrische Körper eine Bohrung auf, deren Durchmesser dem des Lumens entspricht, oder aber kleiner ist; die Bohrung ist so positioniert, daß sich das Lumen in der Bohrung fortsetzt. Die Bohrungswand ist in gleicher Weise wie die Zylinderaußenfläche mit einer Reflexionsschicht überzogen, um einen seitlichen Austritt der Strahlung aus dem zylindrischen Körper in die Bohrung zu verhindern.

Der zylindrische Körper kann sowohl direkt mit seiner proximalen Stirnfläche auf die polierte distale Endfläche eines Faserbündels bzw. der Kombination Fasern/Faserhalter aufgeklebt werden, oder aber in an sich bekannter Weise mittels einer Außenhülse an dem Katheterende befestigt werden. Die Befestigung mit einer Außenhülse ist bevorzugt, da damit eine größere mechanische Stabilität erzielt wird.

Die Reflexionsschicht kann z.B. eine mittels eines Hochvakuumbedampfungsverfahrens aufgetragene Metallschicht sein, wobei die Schichtdicke so bemessen ist, daß die Transmission weitgehend verhindert wird. Bei den meisten Metallen reicht eine Schichtdicke von 10 μm aus. Wird der zylindrische Körper zur Befestigung am Katheterende in eine Außenhülse gefaßt, so daß die Reflexionsschicht nicht mit dem menschlichen Körper in Berührung kommt, kann für die Reflexionsschicht ohne Rücksicht auf eventuelle Körperunverträglichkeiten ein Metall mit hohem Reflexionsvermögen, wie z.B. Ag oder Al, verwendet werden. Zwar absorbieren auch Metalle mit einem hohen Reflexionsvermögen einen nicht ohne weiteres zu vernachlässigenden Anteil der üblicherweise für die beschriebenen medizinischen Anwendungen eingesetzten UV-Strahlung, so daß z.B. eine Beschichtung einer Glasfaser mit einer metallischen Ummantelung wegen der hohen Absorptionsverluste durch die Vielfachreflexionen nicht infrage kommt, der zylindrische Körper ist aber zu kurz, als daß die wenigen Reflexionen zu einer schädigenden Aufheizung des Katheterendes führen könnten.

Grundsätzlich stellt auch die Grenzfläche zwischen einer metallischen Außenhülse und einem mit der Außenhülse mechanisch verpreßten zylindrischen Körper aus einem für die verwendete Laserstrahlung transparenten Material für die Strahlung eine reflektierende Fläche dar; die derzeit bekannten körperverträglichen Legierungen, wie z.B. Edelstahl, die für eine Außenhülse geeignet sind, weisen jedoch ein so hohes Absorptionsvermögen für UV-Strahlung auf, daß auf eine zusätzliche Reflexionsschicht gemäß der Erfindung nicht verzichtet werden kann.

Bevorzugt besteht die Reflexionsschicht aus einem dielektrischen Material, dessen Brechzahl niedriger als die des zylindrischen Körpers ist. Die Schichtdicke ist dabei, wie aus der Glasfasertechnik bekannt, so bemessen, daß die Strahlung durch Totalreflexion am seitlichen Austritt aus dem Körper gehindert wird. Dazu reicht im allgemeinen eine Schichtdicke von wenigstens 3 $\mu$m aus.

Die Beschichtung erfolgt vorzugsweise mittels eines der aus der Glasfasertechnik bekannten CVD-Verfahren. Dies hat den Vorteil, daß ein homogenes, mechanisch und chemisch hochstabiles und körperverträgliches, transparentes optisches Element entsteht. Zur Beschichtung eines Glaszylinders mit einer axialen Bohrung wird z.B. ebenfalls ein CVD-Verfahren verwendet. Aus Gründen der Herstellbarkeit wird dabei ein Quarzglasrohr mit größerem Durchmesser (5-30 mm) beschichtet und anschließend auf den gewünschten Durchmesser ausgezogen. Alternativ werden zwei Glasrohre geeigneter Brechzahl aufeinander aufgeschrumpft (überfangen) bzw. ineinandergesteckt und anschließend auf das gewünschte Maß ausgezogen. Auch geeignete Materialkombinationen für den zylindrischen Körper und die Reflexionsbeschichtung können der Glasfasertechnik entnommen werden. Wegen seiner hohen UV-Durchlässigkeit ist insbesondere Quarzglas geeignet, das zur Erzeugung des Brechzahlunterschiedes zwischen dem zylindrischen Körper und der Reflexionsschicht in an sich bekannter Weise mit brechzahlsenkenden und/oder brechzahlerhöhenden Mitteln dotiert sein kann. Einen Überblick über geeignete Materialkombinationen gibt die nachfolgende Tabelle:

| Lfd.-Nr. | Zyl. Körper | Reflexionsschicht |
|----------|-------------|-------------------|
| 1. | Reines Quarzglas | Quarzglas mit Fluor- und/ oder $B_2O_3$- Dotierung |
| 2. | Quarzglas mit Dotierung von $GeO_2$, $TiO_2$, $Al_2O_3$ und/oder $P_2O_5$ | Reines Quarzglas |
| 3. | dito | wie in Zeile 1 |
| 4. | Reines Quarzglas | Niedrigbrechender Polymerwerkstoff wie: Silikon |
| 5. | optisches Glas 1 (Kombinationen 1 u. 2, | optisches Glas 2 wie für Glasfasern üblich) |

Besonders bevorzugt ist zur Erzielung einer hohen UV-Transmission die Verwendung von reinem Quarzglas für den zylindrischen Körper und F-dotiertem Quarzglas für die Reflexionsschicht. Zwar läßt sich auch mit einer $B_2O_3$-Dotierung eine Brechzahlabsenkung erreichen, F-dotiertes Quarzglas hat aber den Vorteil, daß sein thermischer Ausdehnungskoeffizient dem von reinem Quarzglas eher entspricht.

Neben Quarzglas können aber auch optische Gläser für Zylinder und Reflexionsbeschichtung verwendet werden, wobei geeignete Materialkombinationen für "Kern und Mantel" ebenfalls aus der Glasfaserherstellung bekannt sind. Optische Gläser haben aber den Nachteil, chemisch, thermisch und mechanisch weniger beständig zu sein als Quarzglas.

Für eine axiale Verklebung der Schutzkappe mit dem distalen Ende der Fasern ist insbesondere ein Material geeignet, daß für die verwendete Laserstrahlung hoch-transparent ist und darüber hinaus zur Vermeidung von Reflexionsverlusten bzgl. seiner Brechzahl an das Fasermaterial wie auch an das Schutzkappenmaterial angepaßt ist. Es hat sich gezeigt, daß Hydrosilikate, die üblicherweise zum Verkleben von Glas und Porzellan eingesetzt werden, hierzu besonders geeignet sind. Bevorzugt wird Natriumhydrosilikat als Bindemittel verwendet, dessen Brechzahl nur geringfügig von der des Quarzglases abweicht.

Die Verwendung eines Bindemittels zwischen distaler Faserendfläche und proximaler Zylinderstirnfläche ist auch dann sinnvoll, wenn zur Befestigung am Katheterende zusätzlich ein Außenzylinder verwendet wird, da sich damit die Reflexionsverluste, die auch bei sorgfältig polierten Flächen nicht zu vermeiden sind, herabsetzen lassen.

Die mit der Erfindung erzielbaren Vorteile liegen insbesondere darin, daß die Strahlung, die sich nach Austritt aus den Faserendflächen noch innerhalb des Katheterendes, z.B. in der Schutzkappe, auf Strahlungskegeln ausbreitet und damit auch in Richtung auf die Katheterwand gelenkt wird, in einfacher Weise am seitlichen Austritt aus der Schutzkappe gehindert wird. Dadurch werden nicht nur Verluste an Strahlungsleistung vermieden, sondern auch die Beschädigung des beispielweise zur Befestigung der Schutzkappe in eine Hülse gefaßten Katheterendes durch die bei den bekannten Schutzkappen seitlich in die

EP 0 456 015 A1

Verklebung oder Verpressung austretende und dort absorbierte Strahlung verhindert.

Die erfindungsgemäße Schutzkappe zeichnet sich durch einen einfachen und kompakten Aufbau aus und ist kostengünstig herzustellen. Sie kann leicht an jede Gestalt eines Katheter bzw. eines Katheterlumens angepaßt werden.

Nachfolgend wird die Erfindung anhand der Zeichnungen näher erläutert:

Es zeigen:

Figur 1    in einer schematischen Längsschnittdarstellung eine distale Schutzkappe gemäß der Erfindung, die mittels einer Außenhülse an dem distalen Ende eines Katheters befestigt ist.

Figur 2    in der gleichen Darstellungsweise eine distale Schutzkappe gemäß der Erfindung, welche zur Fortsetzung des Lumens in dem Katheter eine zentrale Bohrung ausweist.

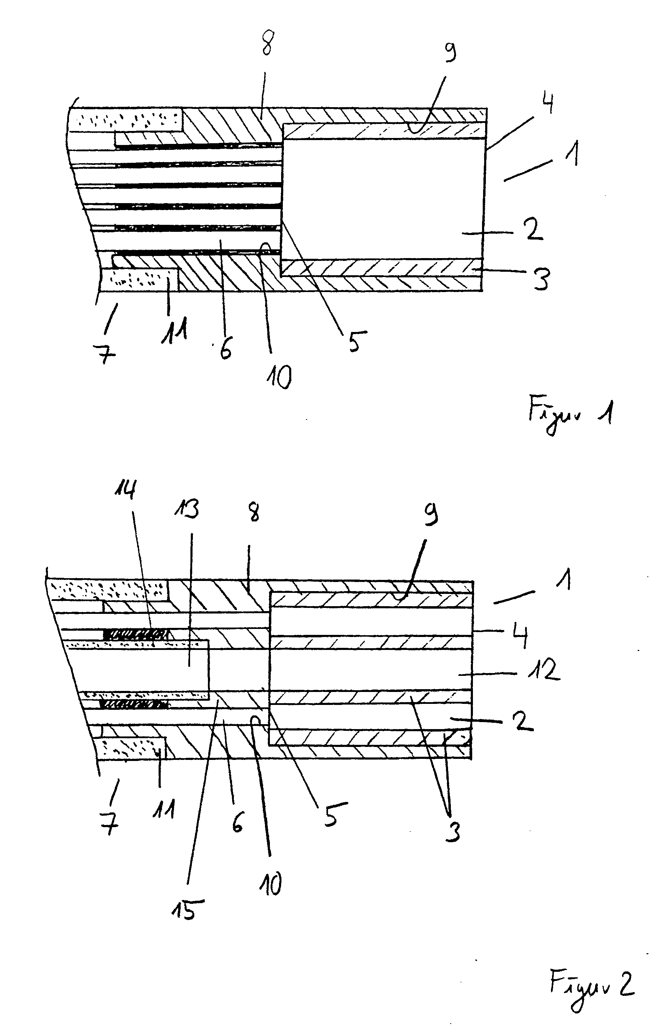

Man erkennt in Figur 1 eine distale Schutzkappe 1, welche aus einem zylindrischen Körper 2 mit einer Reflexionsbeschichtung 3 an der Zylinderaußenfläche besteht. Die distale Stirnfläche 4 stellt die Austrittsoberfläche für die Strahlung dar. Die proximale Stirnfläche 5 des zylindrischen Körpers sitzt auf den distalen Endflächen der Fasern 6 des Katheters 7 auf. Die distalen Enden der Fasern 6 sind miteinander verklebt, die gemeinsame Endfläche ist glatt poliert. Die distale Endfläche des Faserbündels und die proximale Stirnfläche 5 des zylindrischen Körpers 2 können mittels eines für die verwendete Laserstrahlung transparenten Bindemittels miteinander verklebt sein.

Die Schutzkappe 1 ist in an sich bekannter Weise in eine Außenhülse 8 gefaßt. Die Verbindung zwischen der Außenhülse 8 und der Schutzkappe 1 kann durch Verkleben oder mechanisches Verpressen hergestellt sein. Die Außenhülse 8 weist zwei miteinander im Eingriff stehende Bohrungen auf, eine Bohrung 9 mit größerem Durchmesser zur Aufnahme der Schutzkappe 1 und eine Bohrung 10 mit kleinerem Durchmesser zur Aufnahme des fixierten Faserbündelendes. Das Faserbündelende kann an seinem Außenumfang in an sich bekannter Weise mit der Außenhülse 8 , z.B. mittels eines Epoxydklebers, verbunden sein; eine Verklebung der proximalen Stirnfläche der Außenhülse mit dem Ende des Katheterschlauches 11 ist im allgemeinen nicht erforderlich. Weitere Möglichkeiten zur Befestigung einer Hülse am Katheterende sind z.B. in der EP-OS 0 195 375 beschrieben.

In Figur 2 weist die distale Schutzkappe 1 eine zentrale Bohrung 12 auf, die im Eingriff mit dem Lumen 13 im Katheter 7 steht. Die Bohrung 12 ist wie die Außenfläche des zylindrischen Körpers mit einer Reflexionsschicht 3 überzogen. Die Fasern 6 sind auf einem Kreisring um das zentrale Lumen 13 angeordnet. Zur Fixierung sind die Faserenden mit dem das Lumen begrenzenden Innenschlauch 14 verklebt. Zusätzlichen Halt gibt ein Faserhalter 15 in Form einer Hülse, welche auf den Innenschlauch 14 aufgesetzt ist und in einer gemeinsamen distalen Stirnfläche mit den Fasern endet. Der Faserhalter 15 hält und zentriert die Fasern in Bezug zu den Schläuchen (Innen- und Außenschlauch) 7 und 14. Die proximale Stirnfläche 5 kann, wie aus dem Stand der Technik bekannt, mit einer antireflektierenden und die distale Stirnfläche 4 mit einer reflektierenden Schicht überzogen sein. Dies hat den Vorteil, daß einerseits Reflexionsverluste beim Eintritt der Strahlung in die proximale Stirnfläche 5 vermindert werden, andererseits wird die Rückreflexion der Strahlung in den Katheter durch die distale Stirnfläche 4, die zu einer Beschädigung des Katheterendes führen kann, herabgesetzt.

**Patentansprüche**

1.   Distale Schutzkappe für einen Laser-Faser-Katheter zur Entfernung von biologischem Material, mit einem im wesentlichen zylindrischen Körper aus einem für die Laserstrahlung transparenten Material, welcher mit seiner proximalen Stirnfläche die distalen Endflächen der Fasern abdeckt und dessen distale Stirnfläche eine Austrittsoberfläche für die Strahlung festlegt,
     **dadurch gekennzeichnet,**
     daß der zylindrische Körper aus Glas oder Glaskeramik besteht und an seiner Zylinderaußenfläche mit einer Reflexionsschicht versehen ist.

2.   Distale Schutzkappe nach Anspruch 1, für einen Laser-Faser-Katheter mit einem zentralen Lumen,
     **dadurch gekennzeichnet,**
     daß der zylindrische Körper eine zentrale Bohrung aufweist, in die sich das Lumen fortsetzt, und daß die Wand der Bohrung in gleicher Weise wie die Zylinderaußenfläche mit einer Reflexionsschicht überzogen ist.

3.   Distale Schutzkappe nach Anspruch 1 oder 2,
     **dadurch gekennzeichnet,**
     daß die Reflexionsschicht aus einem für die Laserstrahlung transparenten, dielektrischen Material mit

6

einer Brechzahl, die niedriger als die des zylindrischen Körpers ist, besteht, wobei die Schichtdicke in an sich bekannter Weise so bemessen ist, daß die durch die proximale Stirnfläche in den zylindrischen Körper eintretende Strahlung durch Totalreflexion am seitlichen Austritt gehindert wird.

4. Distale Schutzkappe nach Anspruch 3,
   **dadurch gekennzeichnet,**
   daß der zylindrische Körper und die Reflexionsschicht im wesentlichen aus Quarzglas bestehen, wobei zur Erzeugung der Brechzahldifferenz der zylindrische Körper und/oder die Reflexionsbeschichtung in an sich bekannter Weise Dotierungen von F, $B_2O_3$ und/oder $GeO_2$, $TiO_2$, $Al_2O_3$, $P_2O_5$ enthalten.

5. Distale Schutzkappe nach Anspruch 4,
   **dadurch gekennzeichnet,**
   daß der zylindrische Körper aus reinem Quarzglas und die Reflexionsbeschichtung aus fluor-dotiertem Quarzglas bestehen.

6. Distale Schutzkappe nach wenigstens einem der Ansprüche 1-5,
   **dadurch gekennzeichnet,**
   daß der beschichtete zylindrische Körper zur Befestigung am Katheterkörper in an sich bekannter Weise in eine Außenhülse gefaßt ist.

7. Distale Schutzkappe nach wenigstens einem der Ansprüche 1-6,
   **dadurch gekennzeichnet,**
   daß die distalen Faserendflächen mittels eines für die verwendete Laserstrahlung transparenten Bindemittels mit einer an die Brechzahl des Fasermaterials und des zylindrischen Körpers angepaßten Brechzahl mit der proximalen Stirnfläche des im wesentlichen zylindrischen Körpers verklebt sind.

8. Distale Schutzkappe nach Anspruch 7,
   **dadurch gekennzeichnet,**
   daß die distalen Faserendflächen mit der proximalen Stirnfläche mittels Natriumhydrosilikat verklebt sind.

9. Distale Schutzkappe nach wenigstens einem der Ansprüche 3-8,
   **dadurch gekennzeichnet,**
   daß die dielektrische Reflexionsschicht eine mittels eines CVD-Verfahrens aufgetragene Schicht ist.

Figur 1

Figur 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | DE-A-3 833 990 (MBB)<br>* Figur 1a; Spalte 3, Zeilen 28-44 * | 1,3-9 | A 61 B 17/22 |
| Y | | 2 | |
| | – – – | | |
| Y | EP-A-0 338 149 (TRIMEDYNE)<br>* Spalte 5, Zeilen 33-38; Spalte 7, Zeilen 20-28 * | 2 | |
| | – – – | | |
| A | WO-A-9 004 363 (GENERAL HOSPITAL)<br>– – – | | |
| A | EP-A-0 325 836 (BARD)<br>– – – | | |
| A | US-A-4 273 109 (ENDERBY)<br>– – – – – | | |

|  |
|---|
| RECHERCHIERTE SACHGEBIETE (Int. Cl.5) |
| A 61 B<br>A 61 F |

Der vorliegende Recherchenbericht wurde für alle Patentansprüche erstellt

| Recherchenort | Abschlußdatum der Recherche | Prüfer |
|---|---|---|
| Den Haag | 18 Juli 91 | BARTON S.A. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument

&: Mitglied der gleichen Patentfamilie, übereinstimmendes Dokument